(19) Europäisches Patentamt
European Patent Office
Office européen des brevets

(11) **EP 1 083 861 B1**

(12) **EUROPÄISCHE PATENTSCHRIFT**

(45) Veröffentlichungstag und Bekanntmachung des Hinweises auf die Patenterteilung:
**21.09.2005 Patentblatt 2005/38**

(51) Int Cl.⁷: **A61K 7/00**, A61K 7/027, A61K 7/032, A61K 7/32, A61K 7/42

(21) Anmeldenummer: **99931047.7**

(22) Anmeldetag: **09.06.1999**

(86) Internationale Anmeldenummer:
**PCT/EP1999/003979**

(87) Internationale Veröffentlichungsnummer:
**WO 1999/065454 (23.12.1999 Gazette 1999/51)**

(54) **WASSERHALTIGE KOSMETISCHE ODER PHARMAZEUTISCHE STIFTE**

HYDROUS COSMETIC OR PHARMACEUTICAL STICKS

STICKS AQUEUX A USAGE COSMETIQUE OU PHARMACEUTIQUE

(84) Benannte Vertragsstaaten:
**DE ES FR GB IT SE**

(30) Priorität: **12.06.1998 DE 19826118**

(43) Veröffentlichungstag der Anmeldung:
**21.03.2001 Patentblatt 2001/12**

(73) Patentinhaber: **Beiersdorf Aktiengesellschaft**
**20245 Hamburg (DE)**

(72) Erfinder:
• **DIEC, Khiet, Hien**
**D-22523 Hamburg (DE)**
• **GERS-BARLAG, Heinrich**
**D-25495 Kummerfeld (DE)**
• **MÜLLER, Anja**
**D-23843 Rümpel (DE)**
• **SCHREIBER, Jörg**
**D-22087 Hamburg (DE)**

(56) Entgegenhaltungen:
EP-A- 0 522 618          EP-A- 0 748 622
WO-A-98/42301          DE-A- 4 306 068
US-A- 4 873 078

**Beschreibung**

Die vorliegende Erfindung betrifft kosmetische oder pharmazeutische Stifte, welche in Form von W/O-Emulsionen vorliegen und sich durch einen erhöhten Wassergehalt auszeichnen. Insbesondere betrifft die Erfindung dekorative Lippenstifte und Lippenpflegestifte, aber auch desodorierende oder antitranspirierend wirksame Stifte sowie Stiftformulierungen, welche beispielsweise zur Verwendung gegen Akne geeignet sind. Als weitere vorteilhafte Ausführungsformen betrifft die vorliegende Erfindung Sonnenschutzstifte, Augenbrauenstifte, Kajalstifte, Lidschattenstifte, Abdeckstifte und vergleichbare Produkte.

In der Kosmetik gibt es für Stiftformulierungen vorwiegend zwei Anwendungsgebiete: die Bereiche Lippenpflege und Desodorantien.

Die Haut der Lippen besitzt nur eine äußerst dünne Hornschicht. Schweißdrüsen sind auf den Lippen gar nicht, Talgdrüsen nur vereinzelt zu finden. Daher ist die Lippenhaut praktisch frei von Fett und neigt, besonders bei kaltem und trockenem Wetter, zum Austrocknen. Dabei können sich kleine Risse in der Haut bilden, und die Empfindlichkeit der Lippen gegenüber chemischen, physikalischen und mikrobiellen Einwirkungen (z.B. Nahrungsmittel, Sonnenlicht, Herpes-Simplex-Viren) steigt.

Dies zu verhindern, ist eine Aufgabe von Lippenpflegestiften. Diese Produkte enthalten meist zu einem hohen Anteil an Wachsen und Fettkomponenten, die nach dem Auftragen eine abdeckende Schicht über den Lippen ausbilden.

In die Pflegestiftzubereitungen können zusätzlich Wirkstoffe eingearbeitet werden, die die Lippen zusätzlich pflegen oder schützen, wie z. B. Vitamine, Lichtschutzmittel, abdeckende Pigmente usw.

Lippenstifte gehören darüberhinaus zu den am meisten verwendeten dekorativen kosmetischen Mitteln. Ihre praktische Form erlaubt während des Tages ein mehrmaliges Auftragen und Erneuern der Farbe oder des Schutzfilms. In dekorative Lippenstifte können verschiedenste Farbpigmente eingearbeitet werden. Auch diese Stifte enthalten zu hohen Anteilen Wachse und Fettkomponenten, die nach dem Auftragen eine abdeckende Lipidschicht über den Lippen ausbilden. Diese Lipidschicht dient als auf den Lippen haftende Grundlage für die eingearbeiteten Pigmentstoffe, da diese aus mancherlei Gründen nicht ohne eine solche Grundlage auf die Lippen aufgetragen werden können.

Es ist auch möglich, die Eigenschaften der pflegenden und dekorativen Lippenstifte miteinander zu kombinieren, d. h. in dekorative Lippenstifte pflegende oder schützende Substanzen einzuarbeiten.

Der Verbraucher bzw. die Verbraucherin erwartet, daß ein Lippenstift eine saubere, glatte und glänzende Oberfläche besitzt. Zudem dürfen sich weder Öltröpfchen noch Wachskristalle absetzen. Auch die Applikation muß problemlos möglich sein. Lippenstifte sollen sich insbesondere glatt und ohne großen Reibungswiderstand auftragen lassen. Schon bei leichtem Andruck soll der Stift einen gut haftenden Fettfilm an die Lippen abgeben. Der erzielte Film muß die Lippen gut abdecken, möglichst lange haltbar und nicht klebrig sein sowie den Lippen Glanz verleihen. Darüber hinaus müssen sie bruchfest und temperaturbeständig sein, und die Formulierungen dürfen nicht ausölen.

Auch viele dekorative Augenpflegemittel, wie z. B. Augenbrauen- und Kajalstifte, werden in Form von Stiften angeboten. Zum Nachzeichnen und Kolorieren der Augenbrauen werden meist Formulierungen verwendet, bei denen die farbgebenden Pigmente in einer Wachs-/Öl-Basis eingelagert sind. Im allgemeinen wird die fertige Farbstoff-Wachsmasse als Mine extrudiert und in Stiften aus Rotzedernholz konfektioniert. An dekorative Augenpflegestifte stellt die Verbraucherin im Prinzip die gleichen Anforderungen wie an entsprechende Lippenpflegemittel.

Von einem Deodorantstift bzw. einem Antitranspirantstift hingegen wird über die Wirksamkeit hinaus insbesondere erwartet, daß er bei der Anwendung in der Achseln keinen fettigen Eindruck erzeugt.

Sollen kosmetische oder pharmazeutische Stifte bestimmte Wirkstoffe enthalten, ist denkbar, daß die übrigen Bestandteile mit den Wirkstoffen nicht kompatibel sind. Dies ist besonders häufig der Fall, wenn die Verwendung der kosmetischen Stifte als Deo-Stifte vorgesehen ist. Antitranspirierend wirksame Stifte beispielsweise enthalten in der Regel Aluminiumchlorhydrat, welches als kräftige Lewis-Säure gerade für viele Stiftformulierungen nicht verwendbar ist.

Technisch betrachtet, sind insbesondere zwei Arten, Stifte zu formulieren, von besonderer Bedeutung:

Als Lippenstiftgrundmassen sowie im Bereich der dekorativen Augenpflegemittel werden im allgemeinen wasserfreie Fettmischungen aus festen oder halbfesten Wachsen und flüssigen Ölen verwendet. Übliche Grundstoffe des Standes der Technik für stiftförmige Zubereitungen dieser Art sind beispielsweise flüssige Öle (z. B. Paraffinöle, Ricinusöl, Isopropylmyristat), halbfeste Bestandteile (z. B. Vaseline, Lanolin), feste Bestandteile (z. B. Bienenwachs, Ceresin und Mikrokristalline Wachse bzw. Ozokerit) und hochschmelzende Wachse (z. B. Carnaubawachs, Candelillawachs). In Bereich der Lippenpflege sind allerdings auch wasserhaltige Zubereitungen bekannt, welche gelegentlich auch in Form von W/O-Emulsionen vorliegen.

Lippenstifte, Kajalstifte und Augenbrauenstifte des Standes der Technik mit einem Gehalt an Paraffinen und Bienenwachs bzw. Japanwachs sind in *"Kosmetik, Entwicklung Herstellung und Anwendung kosmetischer Mittel"*, *Herausgeber: W. Umbach, Georg Thieme Verlag, Stuttgart - New York, 1995, S. 321 ff*, beschrieben.

Als Grundmassen für desodorierende Stifte hingegen werden in der Regel Seifen-Glykol-Gele verwendet.

Diese entstehen dadurch, daß niedere Glycole und Glycerin in Gegenwart von Natriumstearat klare, transparente Gele bilden, welche zusätzlich Alkohol und Wasser aufnehmen können. Solche Formulierungen sind allerdings nicht mit Aluminiumchlorhydrat verträglich.

Der Stand der Technik hat eine Reihe von Nachteilen. Dazu zählt die Tatsache, daß die Formulierungen in der Regel nur sehr wenig Wasser enthalten können, da ansonsten die Stiftkonsistenz verloren geht. Da aber wasserlösliche Wirkstoffe häufig nicht gut genug fettlöslich sind, lassen sie sich in der Regel nicht in nennenswertem Maße in die kosmetischen Grundlagen einbauen. Ein gewisser Wassergehalt ist darüber hinaus auch deswegen durchaus erwünscht, um die Kompatibilität des kosmetischen Stiftes mit der menschlichen Haut zu erhöhen. Stifte mit hohen Wasseranteilen sind nach dem Stand der Technik aber deshalb nicht machbar, weil Wasser mit der hydrophoben Öl/Wachs/Emulgator-Matrix nicht kompatibel ist.

Eine Aufgabe der vorliegenden Erfindung war es, diesen Mängeln Abhilfe zu schaffen.

Eine weitere Aufgabe der vorliegenden Erfindung war es, Zubereitungen zu entwickeln, welche als Grundlage für kosmetische Desodorantien bzw. Antitranspirantien geeignet sind und die Nachteile des Standes der Technik nicht aufweisen und welche sich weiterhin durch gute Hautverträglichkeit auszeichnen.

Da es dem Lippenbereich praktisch völlig an Pigmenten mangelt, ist dieser gegenüber ultravioletter Strahlung extrem empfindlich. Es empfiehlt sich daher, dem Lippenbereich einen speziellen Schutz gegen UV-Strahlung in Form von entsprechenden stiftförmigen Lichtschutzzubereitungen zukommen zulassen, insbesondere bei erhöhter UV-Exposition, wie beispielsweise im Hochgebirge. Gerade in stiftförmigen Zubereitungen des Standes der Technik werden daher oft anorganischen Pigmente als UV-Absorber bzw. UV-Reflektoren zum Schutze des Lippenbereiches vor UV-Strahlen verwendet. Dabei handelt es sich insbesondere um Oxide des Titans, aber auch gelegentlich des Zinks, Eisens, Zirkoniums, Siliciums, Mangans, Aluminiums, Cers und Mischungen davon, sowie Abwandlungen.

Ein erheblicher Mangel der Formulierungen des Standes der Technik besteht allerdings darin, daß es wegen der niedrigen Wassergehalte an sich akzeptabler Emulsionsstifte praktisch unmöglich war, wasserlösliche UV-Filtersubstanzen in solche Formulierungen einzuarbeiten. Eine weitere Aufgabe der vorliegenden Erfindung war also, diesem Mangel abzuhelfen.

Aus dem DBP 23 35 549 ist ein Verfahren zur Herstellung eines kosmetischen Stiftes auf der Basis einer W/O-Emulsion bekannt. Nach dieser Lehre wird aus einer Polyhydroxyverbindung und einer nichtionogenen, oberflächenaktiven Verbindung ein Gel hergestellt, dieses mit einer kosmetischen Grundlage vermischt und Wasser in die Mischung emulgiert.

Nach diesem Verfahren sind jedoch keine Stifte herzustellen, die über die gestellten universellen Anforderungen an einen kosmetischen Stift verfügen. Da dieses Verfahren darüberhinaus kein Ein-Schritt-Verfahren darstellt, zeichnet es sich durch weitere Nachteile aus.

Die DE-OS 41 28 748 beschreibt kosmetische Stifte, welche dadurch gekennzeichnet sind, daß sie Emulsionen darstellen und als wesentliche Bestandteile Bienenwachs, einen oder mehrere Ester aus einer gesättigten Carbonsäure mit 20 - 40 Kohlenstoffatomen und einem gesättigten Alkohol mit 14 - 34 Kohlenstoffatomen, Wasser sowie gegebenenfalls weitere Lipide und/oder übliche Hilfs- und Zusatzstoffe enthalten. Obwohl diese Zubereitungen zwar vorteilhafte Eigenschaften haben, sind doch noch gewisse Nachteile in Kauf zu nehmen.

Die EP 0 748 622-A1 beschreibt Lippenstiftzubereitungen, welche die Lippen färben sollen und welche eine flüchtige Ölkomponente, ein wasserabweisendes Polymer, welches in der flüchtigen Ölkomponente löslich ist, Pulver und eine nicht-flüchtige Ölkomponente, welche mit der flüchtigen Ölkomponente verträglich ist, enthalten. Mit Pulver werden gemäß der EP 0 748 622-A1 alle pulvrigen Substanzen bezeichnet, welche üblicherweise in Kosmetika eingesetzt werden können. Die beschriebenen Zubereitungen können auch in Form von Emulsionsstiften vorliegen, welche dementsprechend neben einer Ölphase auch 5 Gew.-% Wasser enthalten.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:

- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quaternären Ammonium-Verbindungen.

- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, daß bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch das Zusammenfließen der dispersen Phasen verhindert wird. Die Feststoffpartikel bilden an der Grenzfläche zwischen den beiden flüssigen Phasen sozusagen eine mechanische Barriere gegen die Vereinigung der Flüssigkeitströpfchen.

Eine Möglichkeit, eine Feststoffstabilisierung in einer kosmetischen oder dermatologischen Zubereitung vorzunehmen, ist nach May-Alert (*Pharmazie in unserer Zeit, 15. Jahrg. 1986, Nr. 1, 1-7*) beispielsweise, Emulgatorgemische zu verwenden, die sowohl anionische als auch kationische Tenside enthalten. Da beim Zusammengeben von Anion- und Kationtensiden unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung erreichen. Diese Emulsionen haben allerdings den Nachteil, daß die Menge an üblichen Emulgatoren im Grunde nicht reduziert wird.

Emulsionen, welche durch Feststoffe (zusätzlich) stabilisiert werden, werden üblicherweise auch als Pickering-Emulsionen bezeichnet.

Die Europäische Offenlegungsschrift 0 686 391 beschreibt Emulsionen vom Typ Wasser-in-Öl, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 µm haben. Diese Emulsionen können nach dem oben gesagten als Pickering-Emulsionen bezeichnet werden.

Pickering-Emulsionen des Standes der Technik lassen sich allerdings nicht als Stifte formulieren, da sie in flüssiger bzw. allenfalls zähflüssiger Form vorliegen. Sie werden daher im Bereich der Hautpflege eingesetzt, beispielsweise als Haut- oder Gesichtscrème, Reinigungsmilch, Schutzlotion, Nährcrème und dergleichen mehr.

Eine weitere Aufgabe der vorliegenden Erfindung war es daher, den Stand der Technik um kosmetische oder pharmazeutische Stiftformulierungen zu bereichern, welche in Form von W/O-Emulsionen vorliegen und sich durch einen erhöhten Wassergehalt auszeichnen.

Erstaunlicherweise werden alle diese Aufgaben gelöst durch

emulgatorfreie kosmetische oder dermatologische Stiftzubereitungen, die feindisperse Systeme vom Typ Wasser-in-Öl darstellen, enthaltend

    A) eine Ölphase, die

      einen Gehalt von 10 bis 70 Gew,-%, bezogen auf das Gewicht der Fettphase, an Fett- und/oder Wachskomponenten enthält, welche oberhalb einer Temperatur von 40 °C schmelzen,

    B) eine Wasserphase und

    C) mindestens einen Typ mikronisierter, amphiphiler, anorganischer Pigmente, die

      a) sowohl hydrophile als auch lipophile Eigenschaften zeigen, die also amphiphilen Charakter besitzen und sich daher an der Grenzfläche Wasser/Öl anordnen,

      b) eine mittlere Partikelgröße von weniger als 200 nm haben und die

      c) gewählt werden aus der Gruppe der Metalloxide.

sowie

gegebenenfalls weitere kosmetische oder pharmazeutische Hilfs-, Zusatz- und/oder Wirkstoffe enthaltend.

Der amphiphile Charakter der erfindungsgemäßen anorganischen Pigmente zeigt sich beispielsweise darin, daß diese sowohl in Wasser als auch in Öl dispergierbar sind.

Die erfindungsgemäßen Zubereitungen stellen Abmischungen aus Ölen bzw. öllöslichen Substanzen und Wasser bzw. wasserlöslichen Komponenten dar, die durch den Zusatz der mikronisierten Feststoffpartikel stabilisiert werden und keinen Emulgator im herkömmlichen Sinn enthalten. Eine Erklärungsmöglichkeit für die Stabilität dieser Zubereitungen ist, daß sich die Pigmentpartikel an die Tröpfchen der dispersen Phase anlagern und sozusagen eine mechanische Barriere bilden, die das Koaleszieren, d. h. das Zusammenfließen der Tröpfchen, verhindert.

Die erfindungsgemäßen Zubereitungen sind in jeglicher Hinsicht überaus befriedigende Präparate, die erstaunlicherweise höchst variabel in ihrem Wasser-Fettphasen-Verhältnis sind. Es war insbesondere überraschend, daß die erfindungsgemäßen Stiftzubereitungen einen gegenüber dem Stand der Technik deutlich erhöhten Wassergehalt aufweisen können. Sie zeichnen sich durch exzellente kosmetische Eigenschaften aus und bieten sich daher für den Einsatz in vielen Bereichen der pflegenden und dekorativen Kosmetik an. Für viele Anwendungen ist es insbesondere vorteilhaft, daß die erfindungsgemäßen Zubereitungen frei von Emulgatoren im herkömmlichen Sinne sind. Die erfindungsgemäßen Zubereitungen sind ferner hervorragende Vehikel für verschiedenste Wirkstoffe.

Vorteilhaft ist es, den mittleren Partikeldurchmesser der verwendeten Pigmente zwischen 1 nm und 200 nm, besonders vorteilhaft zwischen 5 nm und 100 nm zu wählen.

Es ist vorteilhaft im Sinne der vorliegenden Erfindung die Metalloxide aus der Gruppe Titandioxid, Zinkoxid, Eisenoxide bzw. Eisenmischoxide, Siliciumdioxid bzw. Silicate (z. B. Talkum) zu wählen, wobei die Metalloxide sowohl einzeln als auch im Gemisch vorliegen können.

Im wesentlichen unerheblich für die vorliegende Erfindung ist es, in welcher der gegebenenfalls natürlich vorkommenden Modifikationen die verwendeten Metalloxide vorliegen.

Der Wasserphasenanteil der erfindungsgemäßen Formulierungen wird vorzugsweise aus dem Bereich von 5 bis 80 Gew.-% gewählt, bezogen auf das Gesamtgewicht der Formulierungen.

Vorteilhaft im Sinne der vorliegenden Erfindung ist es, zur Stabilisierung der Pickering-Emulsionen unbehandelte, nahezu reine Pigmentpartikel zu verwenden, insbesondere solche, welche auch als Farbstoff in der Lebensmittelindustrie und/oder als Absorber von UV-Strahlung in Sonnenschutzmitteln verwendet werden können.

Pickering-Emulsionen im Sinne der vorliegenden Erfindung werden vorteilhaft durch anorganische Pigmente stabilisiert, die oberflächlich wasserabweisend behandelt ("gecoatet") sind, wobei gleichzeitig der amphiphile Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (oberfl.)$$

erzeugt wird. n und m sind dabei nach Belieben einzusetzende stöchiometrische Parameter, R und R' die gewünschten organischen Reste. Besonders vorteilhaft sind $TiO_2$-Pigmente, beispielsweise mit Aluminiumstearat beschichtete.

Eine weitere vorteilhafte Beschichtung der anorganische Pigmente besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Markennamen Eusolex® T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid oder Talkum (Magnesiumsilicat), als auch durch Kombination mehrerer Metalloxidtypen innerhalb einer Zubereitung. Besonders vorteilhaft sind Magnesiumsilicate.

Es ist ferner vorteilhaft, wenngleich nicht zwingend, die erfindungsgemäßen amphiphilen anorganischen Mikropigmente mit weiteren Pigmenten zu kombinieren, beispielsweise mit Titandioxidpigmenten, die mit Octylsilanol beschichtet sind, und/oder mit Siliciumdioxidpartikeln, die oberflächlich wasserabweisend behandelt sind. Zur Kombination geeignete Siliciumdioxidpartikel sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Marken Aerosil® R972 und Aerosil® 200V bei der Firma Degussa erhältlich.

Vorteilhaft in allen vorgenannten Fällen ist es, die Konzentration der erfindungsgemäßen amphiphilen Pigmente größer als 1 Gew.-%, insbesondere zwischen 1 Gew.-% und 30 Gew.-%, besonders vorteilhaft zwischen 2,5 Gew.-% und 7,5 Gew.-%, bezogen auf das Gesamtgewicht der Zubereitungen, zu wählen.

Erfindungsgemäß vorteilhaft werden die hochschmelzenden Fett- und/oder Wachskomponenten aus der Gruppe der pflanzlichen Wachse, tierischen Wachse, Mineralwachse und petrochemischen Wachse gewählt. Erfindungsgemäß günstig sind beispielsweise Candelillawachs, Camaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise Glyceryltribehenat, $C_{16-36}$ Fettsäuretriglycerid und $C_{18-36}$-Fettsäure sowie Montanesterwachse, Sasolwachse, hydrierte Jojobawachse, synthetische oder modifizierte Bienenwachse (z. B. Dimethicon Copolyol Bienenwachs und/oder $C_{30-50}$-Alkyl Bienenwachs), Polyalkylenwachse, Polyethylenglykolwachse, aber auch chemisch modifizierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20-40}$-Alkylstearat, $C_{20-40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen.

Erfindungsgemäße Pickering-Emulsionen können vorteilhaft auch Verdickungsmittel, insbesondere auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974 und/oder Aerosil® R976.

Weitere vorteilhafte Ölverdickungsmittel sind organisch modifizierte Hectorite, wie beispielsweise Quatemium-18-Hektorit oder Stearalkonium Hektorit.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat.

Die erfindungsgemäßen Pickering-Emulsionen können als Grundlage für kosmetische oder dermatologische Formulierungen dienen. Diese können wie üblich zusammengesetzt sein und beispielsweise zur Behandlung und Pflege der Haut, als Lippenpflegeprodukt, als Deoprodukt und als Schmink- bzw. Abschminkprodukt in der dekorativen Kosmetik oder als Lichtschutzpräparat dienen. Es ist gegebenenfalls möglich und vorteilhaft, die erfindungsgemäßen Zusammensetzungen als Grundlage für pharmazeutische Formulierungen zu verwenden.

Zur Anwendung werden die erfindungsgemäßen kosmetischen und dermatologischen Zubereitungen in der für Kosmetika üblichen Weise auf die Haut in ausreichender Menge aufgebracht.

Die erfindungsgemäßen kosmetischen und dermatologischen Zubereitungen können Farbstoffe und/oder Farbpigmente enthalten, insbesondere wenn sie in Form von dekorativen Lippenstiften, Lippenkonturenstiften, Abdeckstiften, Kajalstiften, Augenkonturenstiften und/oder Augenbrauenstiften vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. (Die Stoffe sind nach ihrer Colour Index Number geordnet.)

| Colour Index Number | Deutsche Bezeichnung | CAS-Nr. bzw. Summenformel | Kategorie |
|---|---|---|---|
| 10316 | C-ext. Gelb 1 | 846-70-8 | B |
| 12075 | C-Orange | 3468-63-1 | A |
| 14700 | C-Rot 57 | 4548-53-2 | B |

(fortgesetzt)

| Colour Index Number | Deutsche Bezeichnung | CAS-Nr. bzw. Summenformel | Kategorie |
|---|---|---|---|
| 15510 | C-ext.Orange 8 | 633-96-5 | B |
| 15585 | C-Rot 55 | 2092-56-0 | B |
| 15585:1 | C-Rot 55 | 5160-02-1 | B |
| 15800:1 | C-ext. Rot 57 | 6371-76-2 | A |
| 15850 | Lithol Rubin 8 | 5858-81-1 | A |
| 15850:1 | C-Rot 12 | 5281-04-9 | A |
| 15880:1 | C-ext. Rot 61 | 6417-83-0 | A |
| 15980 | C-Orange 9 | $C_{16}H_{10}N_2O_7S_2 \cdot 2Na$ | A |
| 15985 | C-Orange 10 | 2783-94-0 | A |
| 16035 | C-Rot 60 | 29956-17-6 | A |
| 17200 | C-Rot 58 | $C_{16}H_{13}N_3O_7S_2 \cdot 2Na$ | A |
| 19140 | C-Gelb 10 | 1934-21-0 | A |
| 20170 | C-ext. Braun 4 | 1320-07-6 | A |
|  |  | 6371-84-2 |  |
| 26100 | C-ext. Rot 56 | 85-86-9 | A |
| 42053 | C-Grün 12 | $C_{37}H_{36}N_2O_{10}S_3 \cdot 2Na$ | A |
| 42090 | C-Blau 21 | 2650-18-2 | A |
| 42090 | C-Blau 21 | 2650-18-2 | A |
|  | (Ammonium Salz) | 6371-85-3 |  |
|  |  | 37307-56-5 |  |
| 45170 | C-Rot 59 | 81-88-9 | B |
| 45170:1 | (Rhodamin B-stearat) | $C_{28}H_{31}N_2O_3 \cdot C_{18}H_{35}O_2$ | B |
| 45370:1 | C-Rot 27 | $C_{20}H_{10}Br_2O_5$ | A |
| 45380 | C-Rot 30 | 17372-87-1 | A |
| 45380:2 | Tetrabromfluoreszein | 15086-94-9 | A |
| 45410 | C-Rot 34 | 18472-87-2 | A |
| 45410:1 | Tetrabromtetrachlorfluoreszein | 13473-26-2 | A |
| 45425 | C-Rot 35 | $C_{20}H_{10}I_2O_5 \cdot 2Na$ | A |
| 45425:1 | Fluoreszein-Gemisch | 518-40-7 | A |
|  |  | 38577-97-8 |  |
| 47000 | C-ext. Gelb 23 | 8003-22-3 | B |
| 47005 | C-Gelb 11 | 8004-92-0 | A |
| 59040 | C-ext. Gelb 24 | 6358-69-6 | A |
| 60725 | C-ext. Violett 18 | 81-48-1 | A |
| 61565 | C-Grün 10 | 128-80-3 | A |
| 61570 | C-Grün 11 | 4403-90-1 | A |
| 73360 | C-Rot 28 | 2379-74-0 | A |
| 75120 | C-Orange 12 | 8015-67-6 | A |
| 75130 | C-Orange 11 | 7235-40-7 | A |
| 75170 | Guanin | 68-94-0 | A |
|  |  | 73-40-50 |  |
| 75470 | C-Rot 50 | $C_{22}H_{20}O_{13}$ | A |
| 75480 | Henna | $C_{10}H_6O_3$ (Lawson) | A |
| 75810 | C-Grün 8 | 11006-34-1 | A |
| 75810 | C-Grün 7 | 479-61-8 | A |
|  |  | 519-62-0 | A |
| 77000 | C-Pigment 1 | Al | A |
| 77007 | C-Blau 16 | 57455-37-5 | A |
| 77019 | C-Weiß 11 | 12001-26-2 | A |

(fortgesetzt)

| Colour Index Number | Deutsche Bezeichnung | CAS-Nr. bzw. Summenformel | Kategorie |
|---|---|---|---|
| 77288 | C-Grün 9 | 1308-38-9 | A |
| 77289 | C-Grün 14 | 12001-99-9 | A |
| 77400 | Bronze | 7440-50-8 | A |
| 77491 | C-Rot 45 | 1309-37-1 | A |
| 77492 | C-Braun 3 (C-Gelb 8) | $Fe_2O_3FeO(OH)$ | A |
| 77499 | C-Schwarz 5 | $Fe_3O_4$ | A |
| 77510/20 | C-Blau 17 | $C_6FeN_6 \cdot 4/3$ Fe | A |
| 77742 | C-Violett 11 | 10101-66-3 | A |
| 77820 | C-Pigment 2 | 7440-22-4 | A |
| 77891 | C-Weiß 7 | 13463-67-7 ($TiO_2$) | A |
| 77947 | C-Weiß 8 | 1314-13-2 | A |

Die Kategorien (A) und (B) sind folgendermaßen definiert:

**(A)**

Der Farbstoff kann verwendet werden für die Herstellung von oral zu verabreichenden Zubereitungen, wie z. B. Lippenpflegemittel, für Zubereitungen, die im Bereich der Augen zur Anwendung kommen, für externe Zubereitungen (Cremes, Lotionen, Puder), für Zubereitungen, welche wieder abgespült werden (Rinse-off [RO]-Products, wie z. B. Haarwaschmittel, Haarkonditioniermittel usw.), für Haarfärbemittel, für Nagelpflegemittel.
Eine Kennzeichnung der Farbstoffe ist nicht erforderlich.

**(B)**

Der Farbstoff kann verwendet werden für die Herstellung von Zubereitungen, die unter (A) gelistet werden, jedoch nicht für die Herstellung von Präparaten, die im Bereich der Augen zur Anwendung kommen.
Die Farbstoffe und Pigmente können sowohl einzeln als auch im Gemisch vorliegen sowie gegenseitig miteinander beschichtet sein, wobei durch unterschiedliche Beschichtungsdikken im allgemeinen verschiedene Farbeffekte hervorgerufen werden.
Die Liste der genannten Farbstoffe und Farbpigmente, die in den erfindungsgemäßen Pikkering-Emulsionsstiften verwendet werden können, soll selbstverständlich nicht limitierend sein.
Die erfindungsgemäßen kosmetischen und dermatologischen Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Pigmente, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.
Eine erstaunliche Eigenschaft der erfindungsgemäßen Zubereitungen ist, daß diese sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.
Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die erfindungsgemäßen Stiftzubereitungen mit einem Gehalt an einem oder mehreren Antioxidantien zu versehen, wenn die Zubereitungen einem anderen Zweck dienen sollen, z. B. als Desodorantien oder Sonnenschutzmittel.
Besonders vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Lino-leyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Die Liste der genannten Wirkstoffe bzw. Wirkstoffkombinationen, die in den erfindungsgemäßen Pickering-Emulsionsstiften verwendet werden können, soll selbstverständlich nicht limitierend sein.

Günstig sind auch kosmetische und dermatologische Stiftzubereitungen, die in der Form eines Sonnenschutzmittels vorliegen. Vorzugsweise enthalten diese mindestens eine UV-A-Filtersubstanz und/oder mindestens eine UV-B-Filtersubstanz und/oder mindestens ein weiteres anorganisches Pigment aus der Gruppe der Oxide des Eisens, Zirkoniums, Siliciums, Mangans, Aluminiums, Cers und Mischungen davon sowie Abwandlungen, bei denen die Oxide die aktiven Agentien sind.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So können beispielsweise in pflegende Lippenstifte UV-A- bzw. UV-B-Filtersubstanzen eingearbeitet werden.

Vorteilhaft können erfindungsgemäße Zubereitungen Substanzen enthalten, die UV-Strahlung im UV-B-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Stifte zur Verfügung zu stellen, die die Haut vor dem gesamten Bereich der ultravioletten Strahlung schützen.

Enthalten die erfindungsgemäßen Emulsionen UV-B-Filtersubstanzen, können diese öllöslich oder wasserlöslich sein. Erfindungsgemäß vorteilhafte öllösliche UV-B-Filter sind z.B.:

- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;

- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- hinsichtlich der C3-Achse des Triazingrundkörpers symmetrisch oder unsymmetrisch substituierte Triazinderivate, vorzugsweise 4,4',4''-(1,3,5-Triazin-2,4,6-triyltriimino)-tris-benzoesäure-tris(2-ethylhexylester) (symmetrisch) sowie 2,4-Bis-{[4-(2-Ethyl-hexyloxy)-2-hydroxy]-phenyl}-6-(4-methoxyphenyl)-1,3,5-triazin, 2,4-Bis-{[4-(3-sulfonato)-2-hydroxy-propyloxy)-2-hydroxy]-phenyl}-6-(4-methoxyphenyl)-1,3,5-triazin Natriumsalz, 2,4-Bis-{[4-(3-(2-Propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethyl-carboxyl)-phenylamino]-1,3,5-triazin, 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, 2,4-Bis-{[4-(2-ethyl-hexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2''-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2''-methyl-propyloxy)-2-hydroxy]-phenyl}-6-(4-methoxyphenyl)-1,3,5-triazin (unsymmetrisch),
- Benzotriazolderivate, vorzugsweise 2,2'-Methylen-bis-(6-(2H-benzotriazol-2-yl)-4-(1,1,3,3-tetramethylbutyl)-phenol)
- sowie an Polymere gebundene UV-Filter.

Vorteilhafte wasserlösliche UV-B-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bomylidenmethyl)sulfonsäure und ihre Salze.

Die Liste der genannten UV-B-Filter, die in den erfindungsgemäßen Pickering-Emulsionen verwendet werden können, soll selbstverständlich nicht limitierend sein.

Es kann auch von Vorteil sein, in erfindungsgemäßen Pickering-Emulsionen UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die UV-A-Filter enthalten, sind Gegenstand der Erfindung. Es können die für die UV-B-Kombination verwendeten Mengen eingesetzt werden.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Deo-Stifte betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen W/O-Emulsionsstifte eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Famesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-19516 705 beschriebenen Wirkstoffe bzw. Wirkstoffkombinationen. Auch Natriumhydrogencarbonat ist vorteilhaft zu verwenden.

Die Liste der genannten Wirkstoffe bzw. Wirkstoffkombinationen, die in den erfindungsgemäßen Pickering-Emulsionsstiften verwendet werden können, soll selbstverständlich nicht limitierend sein.

Die erfindungsgemäßen kosmetischen Desodorantien können in Form von aus normalen Behältern auftragbaren wasserhaltigen, kosmetischen Stifte vorliegen.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Die erfindungsgemäßen Stifte sind ferner hervorragende Vehikel für dermatologische Wirkstoffe. Insbesondere eignen sie sich als Träger für gegen Akne wirksame Substanzen. Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes*).

Daher ist es vorteilhaft, den erfindungsgemäßen Zubereitungen gegen Akne wirksame Substanzen zuzugeben, die beispielsweise gegen *Propionibacterium* acnes wirksam sind (etwa solche, die in DE-OS 42 29 707, DE-OS 43 05 069, DE-OS 43 07 976, DE-OS 43 37 711, DE-OS 43 29 379 beschrieben werden) aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol ($\alpha$-Octadecylglycerylether), Selachylalkohol ($\alpha$-9-Octadecenylglycerylether), Chimylalkohol ($\alpha$-Hexadecylglycerylether) und/oder Bisabolol sowie Antibiotika und/oder Keratolytika.

Keratolytika sind Stoffe, die verhornte Haut (wie z. B. Warzen, Hühneraugen, Schwielen und dergleichen mehr) erweichen, damit sich diese leichter entfernen läßt oder damit sie abfällt bzw. sich auflöst.

Alle gängigen gegen Akne wirksamen Substanzen können vorteilhaft genutzt werden, insbesondere Benzoylperoxid, Bituminosulfonate (Ammonium-, Natrium- und Calcium-Salze von Schieferöl-Sulfonsäuren), Salicylsäure (2-Hydroxybenzoesäure), Miconazol (1-[2-(2,4-Dichlorbenzyloxy)-2-(2,4-dichlorphenyl)-ethyl]-imidazol) und Derivate, Adapalen (6-[3-(1-Adamantyl)-4-methoxyphenyl]-2-naphthoesäure), Azelainsäure (Nonandisäure), Mesulfen (2,7-Dimethylthianthren, $C_{14}H_{12}S_2$) sowie Aluminiumoxid, Zinkoxid und/oder feinverteilter Schwefel.

Die Menge der Antiaknemittel (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Erfindungsgemäße W/O-Pickering-Emulsionsstifte sind erhältlich, indem man zunächst die Pigmente in der Fettphase dispergiert und die Fettphase anschließend mit der Wasserphase vereinigt.

Gegenstand der Erfindung ist daher auch ein Verfahren zur Herstellung der erfindungsgemäßen Pickering-Emulsionsstifte, das dadurch gekennzeichnet ist, daß man die amphiphilen anorganischen Pigmente in an sich bekannter Weise in der Fettphase, welche 10 bis 70 Gew.-%, bezogen auf das Gewicht der Fettphase, an Fett- und/oder Wachskomponenten enthält, welche oberhalb einer Temperatur von 40 °C schmelzen, und gewünschtenfalls kosmetische oder pharmazeutische Hilfs-, Zusatz- und/oder Wirkstoffe enthält, bei gleichmäßigem Rühren und gegebenenfalls unter Erwärmen dispergiert und während des Homogenisiervorgangs die Wasserphase, welche gewünschtenfalls ebenfalls kosmetische oder pharmazeutische Hilfs-, Zusatz- und/oder Wirkstoffe enthält, mit der Fettphase vermischt.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Beispiele:

| | Lippenpflege-stift | Sonnenschutz-stift |
|---|---|---|
| Caprylic/Capric Triglycerid | 10 | |
| Octyldodecanol | | 10 |
| Dicaprylylether | 15 | |
| C$_{12-15}$ Alkylbenzoat | | 15 |
| Ricinusöl | 5 | |
| Octyltriazon | | 2 |
| 4-Methylbenzyliden Campher | | 2 |
| Butylmethoxydibenzoylmethan | | 2 |
| Vitamin E-Acetat | | 0,5 |
| Konservierungsmittel | 0,5 | 0,5 |
| Kesterwachs K82H (C$_{20-40}$ Alkylstearat) | 10 | 12 |
| Kesterwachs K80P (C$_{16-38}$ Alkylhydroxystearoylstearat) | | 2 |
| Silica (Aerosil®R972) | 1 | 0,5 |
| Eusolex® T2000 | 4 | 2 |
| Zinkoxid | | 2 |
| Glycerin | 5 | 10 |
| Phenylbenzimidazolsulfonsäure | | 2 |
| NaOH (45%ige Lösung in Wasser) | | 0,7 |
| Wasser | ad 100 | ad 100 |

| | Lippenpflege-stift | dekorativer Lippenstift |
|---|---|---|
| Caprylic/Capric Triglycerid | | 10 |
| Octyldodecanol | 10 | 10 |
| Dicaprylylether | | 10 |
| C$_{12-15}$ Alkylbenzoat | 12 | |
| Mineralöl | 10 | |
| Cycolmethicon | 1 | |
| Vitamin E-Acetat | | 0,5 |
| Konservierungsmittel | 0,5 | 0,5 |
| Kesterwachs K82H (C$_{20-40}$ Alkylstearat) | | 8 |
| Bienenwachs | 4 | |
| Carnauberwachs | | 4 |
| Ozokerit | 12 | |
| Vaseline | 10 | 5 |
| Bentone 38 (Quaternium-18 Hectorit) | | 1 |
| Silica (Aerosil®R972) | 0,5 | |
| Eusolex® T2000 | 2 | 2 |
| Zinkoxid | 2 | 5 |
| Farbpigmente | | 5 |
| Glycerin | 5 | 3 |
| Wasser | ad 100 | ad 100 |

|  | Deostift | Augenbrauen-stift |
|---|---|---|
| Caprylic/Capric Triglycerid | 15 | 5 |
| Octyldodecanol | 10 | |
| Dicaprylylether | 5 | |
| $C_{12-15}$ Alkylbenzoat | | 5 |
| Mineralöl | | 5 |
| Ricinusöl | 3 | |
| Glycerylmonolaurat | 0,5 | |
| Konservierungsmittel | | 0,5 |
| Kesterwachs K82H ($C_{20-40}$ Alkylstearat) | 15 | 5 |
| Bienenwachs | | 10 |
| Carnauberwachs | | 10 |
| Bentone 38 (Quaternium-18 Hectorit) | 1 | |
| Eusolex® T2000 | 4 | 2 |
| Zinkoxid | | 4 |
| Farbpigmente | | 20 |
| Glycerin | 5 | 3 |
| Aluchlorhydrat | 5 | |
| Wasser | ad 100 | ad 100 |

**Patentansprüche**

1. Emulgatorfreie kosmetische oder dermatologische Stiftzubereitungen, die feindisperse Systeme vom Typ Wasser-in-Öl darstellen, enthaltend

A) eine Ölphase, die
einen Gehalt von 10 bis 70 Gew.-%, bezogen auf das Gewicht der Fettphase, an Fett- und/oder Wachskomponenten enthält, welche oberhalb einer Temperatur von 40 °C schmelzen,
B) eine Wasserphase und
C) mindestens einen Typ mikronisierter, anorganischer Pigmente, die

a) eine mittlere Partikelgröße von weniger als 200 nm haben und deren Partikel
b) sowohl hydrophile als auch lipophile Eigenschaften zeigen, welche also amphiphilen Charakter besitzen und sowohl in Wasser als auch in Öl dispergierbar sind und die
c) gewählt werden aus der Gruppe der Metalloxide, die
d) gegebenenfalls oberflächlich beschichtet sind.

2. Zubereitungen nach Anspruch 1, **dadurch gekennzeichnet, daß** weitere kosmetische oder pharmazeutische Hilfs-, Zusatz- und/oder Wirkstoffe enthalten sind.

3. Zubereitungen nach einem der vorhergehenden Ansprüche, **dadurch gekennzeichnet, daß** der Gehalt der verwendeten anorganischen Pigmente zwischen 0,1 Gew,-% und 30 Gew.-% ist, bezogen auf das Gesamtgewicht der Zubereitungen.

4. Zubereitungen nach einem der vorhergehenden Ansprüche, **dadurch gekennzeichnet, daß** der Partikeldurchmesser der verwendeten anorganischen Pigmente zwischen 5 nm und 100 nm liegt.

5. Zubereitungen nach einem der vorhergehenden Ansprüche, **dadurch gekennzeichnet, daß** das oder die anorganischen Pigmente gewählt werden aus der Gruppe Titandioxid, Zinkoxid und Talkum sind.

6. Zubereitungen nach einem der vorhergehenden Ansprüche, **dadurch gekennzeichnet, daß** die verwendeten anorganischen Pigmente oberflächlich wasserabweisend behandelt sind, wobei der amphiphile Charakter der Pig-

13

mente gebildet wird bzw, erhalten bleibt.

**7.** Zubereitungen nach einem der vorhergehenden Ansprüche, **dadurch gekennzeichnet, daß** die verwendeten anorganischen Pigmente mit Dimethylpolysiloxan und/oder Silicagel und/oder Aluminiumhydroxid und/oder Siliziumdioxid beschichtet sind.

**8.** Zubereitungen nach einem der vorhergehenden Ansprüche, **dadurch gekennzeichnet, daß** sie zusätzlich einen oder mehrere Farbstoffe und/oder Farbpigmente enthalten.

**9.** Zubereitungen nach einem der vorhergehenden Ansprüche, **dadurch gekennzeichnet, daß** sie einen oder mehrere Zusatz- oder Wirkstoffe enthalten, gewählt aus der Gruppe der Antioxidantien und/oder UV-Schutzmittel.

**10.** Zubereitungen nach einem der vorhergehenden Ansprüche, **dadurch gekennzeichnet, daß** sie einen oder mehrere Zusatz- oder Wirkstoffe enthalten, gewählt aus der Gruppe der Adstringentien und/oder antimikrobiell wirksamen Stoffe und/oder gegen Akne wirksamen Stoffe.

**11.** Verfahren zur Herstellung von Pickering-Emulsionsstiften nach Anspruch 1, **dadurch gekennzeichnet, daß** amphiphile anorganischen Pigmente in an sich bekannter Weise in der Fettphase, welche einen Gehalt von 10 bis 70 Gew, %, bezogen auf das Gewicht der Fettphase, an Fett- und/oder Wachskomponenten enthält, welche oberhalb einer Temperatur von 40 °C schmelzen, und gewünschtenfalls kosmetische oder pharmazeutische Hilfs-, Zusatz- und/oder Wirkstoffe enthält, bei gleichmäßigem Rühren und gegebenenfalls unter Erwärmen dispergiert werden und während des Homogenisiervorgangs die Wasserphase, welche gewünschtenfalls ebenfalls kosmetische oder pharmazeutische Hilfs-, Zusatz- und/oder Wirkstoffe enthält, mit der Fettphase vermischt wird.

## Claims

**1.** Emulsifier-free cosmetic or dermatological stick preparations which are finely disperse systems of the water-in-oil type, comprising

    A) an oil phase which
      comprises from 10 to 70% by weight, based on the weight of the fatty phase, of fatty and/or wax components which melt above a temperature of 40°C,
    B) an aqueous phase and
    C) at least one type of micronized, inorganic pigments which

      a) have an average particle size of less than 200 nm and whose particles
      b) display both hydrophilic and also lipophilic properties, which thus have amphiphilic character, and are dispersible both in water and in oil, and which
      c) are chosen from the group consisting of metal oxides which
      d) optionally have been coated on the surface.

**2.** Preparations according to Claim 1, **characterized in that** further cosmetic or pharmaceutical auxiliaries, additives and/or active ingredients are present.

**3.** Preparations according to one of the preceding claims, **characterized in that** the content of inorganic pigments used is between 0.1 % by weight and 30% by weight, based on the total weight of the preparations.

**4.** Preparations according to one of the preceding claims, **characterized in that** the particle diameter of the organic pigments used is between 5 nm and 100 nm.

**5.** Preparations according to one of the preceding claims, **characterized in that** the inorganic pigment or pigments are chosen from the group consisting of titanium dioxide, zinc oxide and talc.

**6.** Preparations according to one of the preceding claims, **characterized in that** the inorganic pigments used have been surface-treated to repel water, where the amphiphilic character of the pigments is formed or retained.

**7.** Preparations according to one of the preceding claims, **characterized in that** the inorganic pigments used have been coated with dimethylpolysiloxane and/or silica gel and/or aluminium hydroxide and/or silicon dioxide.

8.  Preparations according to one of the preceding claims, **characterized in that** they additionally comprise one or more dyes and/or coloured pigments.

9.  Preparations according to one of the preceding claims, **characterized in that** they comprise one or more additives or active ingredients chosen from the group consisting of antioxidants and/or UV protectants.

10. Preparations according to one of the preceding claims, **characterized in that** they comprise one or more additives or active ingredients chosen from the group consisting of astringents and/or antimicrobial substances and/or substances which are effective against acne.

11. Method of preparing Pickering emulsion sticks according to Claim 1, **characterized in that** amphiphilic inorganic pigments are dispersed in a manner known per se in the fatty phase, which comprises from 10 to 70% by weight, based on the weight of the fatty phase, of fatty and/or wax components which melt above a temperature of 40°C, and, if desired, cosmetic or pharmaceutical auxiliaries, additives and/or active ingredients, with uniform stirring and optionally with heating, and during the homogenization operation the aqueous phase, which, if desired, also comprises cosmetic or pharmaceutical auxiliaries, additives and/or active ingredients, is mixed with the fatty phase.

**Revendications**

1.  Préparations en bâtons cosmétiques ou dermatologiques, qui représentent des systèmes finement dispersés du type eau-dans-huile, contenant

    A) une phase huileuse qui
        a une teneur de 10 à 70 % en poids, par rapport au poids de la phase lipidique, en composants de type graisse et/ou cire qui fondent au-dessus d'une température de 40°C,
    B) une phase aqueuse et
    C) au moins un type de pigments minéraux micronisés qui

        a) ont une taille moyenne de particule de moins de 200 nm et dont les particules
        b) présentent aussi bien des propriétés hydrophiles que des propriétés lipophiles, qui ont donc un caractère amphiphile et sont dispersables aussi bien dans l'eau que dans l'huile et qui
        c) sont choisis dans le groupe des oxydes métalliques, qui
        d) sont éventuellement enrobés en surface.

2.  Préparations selon la revendication 1, **caractérisées en ce que** d'autres adjuvants, additifs et/ou actifs cosmétiques ou pharmaceutiques sont contenus.

3.  Préparations selon l'une quelconque des revendications précédentes, **caractérisées en ce que** la teneur en les pigments minéraux utilisés est comprise entre 0,1 % en poids et 30 % en poids, par rapport au poids total des préparations.

4.  Préparations selon l'une quelconque des revendications précédentes, **caractérisées en ce que** le diamètre de particule des pigments minéraux utilisés est compris entre 5 nm et 100 nm.

5.  Préparations selon l'une quelconque des revendications précédentes, **caractérisées en ce que** le ou les pigments minéraux sont choisis parmi le dioxyde de titane, l'oxyde de zinc et le talc.

6.  Préparations selon l'une quelconque des revendications précédentes, **caractérisées en ce que** les pigments minéraux utilisés sont traités en surface par un traitement hydrophobe, de sorte que le caractère amphiphile des pigments est conféré ou est maintenu.

7.  Préparations selon l'une quelconque des revendications précédentes, **caractérisées en ce que** les pigments minéraux utilisés sont enrobés de diméthylpolysiloxane et/ou de gel de silice et/ou d'hydroxyde d'aluminium et/ou de dioxyde de silicium.

8.  Préparations selon l'une quelconque des revendications précédentes, **caractérisées en ce qu'**elles contiennent additionnellement un ou plusieurs colorants et/ou pigments colorés.

9.  Préparations selon l'une quelconque des revendications précédentes, **caractérisées en ce qu'**elles contiennent un ou plusieurs actifs ou additifs choisis dans le groupe des antioxydants et/ou des photoprotecteurs UV.

10. Préparations selon l'une quelconque des revendications précédentes, **caractérisées en ce qu'**elles contiennent un ou plusieurs actifs ou additifs choisis dans le groupe des astringents et/ou des substances à activité antimicrobienne et/ou des substances actives contre l'acné.

11. Procédé pour la fabrication de bâtons à base d'émulsions de Pickering selon la revendication 1, **caractérisé en ce qu'**on disperse des pigments minéraux amphiphiles, d'une façon connue en soi, dans la phase lipidique qui contient de 10 à 70 % en poids, par rapport au poids de la phase lipidique, de composants de type graisse et/ou cire qui fondent au-dessus d'une température de 40°C, et, si on le désire, des adjuvants, additifs et/ou actifs cosmétiques ou pharmaceutiques, avec agitation constante et éventuellement en chauffant, et pendant le processus d'homogénéisation on mélange avec la phase lipidique la phase aqueuse qui, si on le désire, contient également des adjuvants, additifs et/ou actifs cosmétiques ou pharmaceutiques.